# EUROPEAN PATENT APPLICATION

(11) **EP 4 790 129 A1**
(43) Date of publication of application: **12.08.2026**
(21) Application number: 25305175.9
(22) Date of filing: 06.02.2025
(51) Int. Cl.: C12M 1/32, C12M 1/00, C12M 3/06

(54) **CELL CULTURE SYSTEM COMPRISING A MULTI-WELL CULTURE PLATE AND A MICROFLUIDIC DEVICE**

(71) Applicant: Cherry Biotech SAS, 93100 Montreuil (FR)
(72) Inventor: FASSINI, Dario, 93100 Montreuil (FR); ORTEGA, Alberto, 93100 Montreuil (FR); DAWSON, Harry, 93100 Montreuil (FR); CADOUX, Mathilde, 93100 Montreuil (FR); CRAMER, Jérémy, 93100 Montreuil (FR)
(74) Representative: Vidon Brevets & Stratégie

(57) **Abstract**

The invention concerns a cell culture system that comprises a multi-well cell culture plate (10) comprising at least one well (110) having a predetermined longitudinal shape delimited by an inner peripheral edge and terminating in a bottom surface (111) which is used for culture of a biological entity, and a microfluidic device (20) configured to be mounted on the multi-well cell culture plate (10) to create at least one closed chamber in which a volume of liquid cell culture medium flows from a liquid cell culture medium inlet opening (215) into the at least one closed chamber to a liquid cell culture medium outlet opening (216) into the at least one closed chamber in order to culture the biological entity, the microfluidic device (20) comprises a manifold containing at least one nozzle (210) having a predefined longitudinal shape extending along a longitudinal axis delimited by an outer peripheral edge (211) and terminating in an insertion base (212) substantially perpendicular to the longitudinal axis configured to be inserted into the at least one well (110) to form the at least one closed chamber having a height defined between the insertion base (212) of the at least one nozzle (210) and the bottom surface (111) of the at least one well (110).

## Description

### Technical field

The invention relates to the field of *in vitro* culture of cells, tissues or organoids in culture plates, in particular so-called multi-well plates, or in fluidic devices. In particular, the invention relates to the control of the environmental conditions of *in vitro* culture within a confined space such as cell culture plates, or in fluidic devices. More particularly, the invention relates to a cell culture system configured to control both the fluidic perfusion into multi-well cell culture plate for *in vitro* cell culture and also the shear stress applied to a biological entity contained within the multi-well cell culture plate.

### Previous technique

*In vitro* cultures of cells, or of cellular assembly such as tissue culture, of spheroids or even more recently of organoids, i.e. of three-dimensional multicellular structures which reproduce *in vitro* the micro-anatomy or physiology of an organ, are today increasingly used as a tool for the toxicological evaluation of substances, in particular pharmacological or cosmetic, or biotherapies, such as those based on immune cells.

These *in vitro* models are used, particularly in pharmaceutical research, because they represent a promising alternative to *in vivo* models, i.e. animal experimentation, which presents both economic and ethical issues on an international scale.

Thus, to be able to carry out controlled biological experiments using these *in vitro* models and to analyse therapeutic compounds, cosmetics or potentially dangerous substances, it is essential to automate the maintenance of cell culture parameters and the fine control of selective environmental changes in confined cellular culture environments, in order to replicate *in vivo* physiopathology.

Microfluidic cell culture devices, understood as the cultivation of cells in chambers, or reservoirs, connected and fed by microchannels, for example in a manifold, characterized by fluidic volumes between 1pL and 100mL, are an important technology for drug screening, tissue culture, detection of toxicity and biological research. Such devices improve cell culture conditions, permit multiple cell types to communicate, as well as enable better control of chemical and physical mechanisms such as diffusion and shear-stress. Moreover, such devices provide better quality experimental data, while economising reagent consumption and reducing costs.

*In vitro* models are of growing interest for both academic and pharmaceutical research as they represent an ethical, relatively low-cost, and more relevant approach in respect to traditional animal and 2D cell culture models. These *in vitro* models offer increased complexity by being able to recapitulate *in vivo* physiological phenomena that cannot be replicated in simple 2D *in vitro* models. Mechanical forces, often neglected in these 2D *in vitro* models, play an essential role in the behaviour of biological entities being cultured by activating downstream signalling pathways through a process termed mechano-transduction. To date however, such devices have been highly challenging to integrate within industrial pipelines. As a result, there is growing interest in systems using industry-standard multi-well plates to provide a simple means of device integration.

There is therefore a need for providing Micro-Physiological Systems (commonly known as MPS in English) comprising at least a multi-well plate comprising a well configured to culture a biological entity. The at least one multi-well plate allows the biophysical parameters within the well to be controlled in order to improve the representation of the *in vivo* environment, thus providing more appropriate *in vitro* models for toxicity testing of drugs applied to the biological entity being cultured.

For example, the well is configured to contain tissue sample and/or an extra-cellular matrix coating, with cells embedded within and/or above.

Among biomechanical forces exerted inside the well on the biological entity being cultured and controlled by MPS devices, shear stress plays a critical role on the cells of the biological entity being cultured through mimicking *in vivo* conditions for endothelial, stem and cartilage cells. Shear stress is defined as the force per unit area exerted by fluid flow, such as blood or culture media, across a cell surface.

This shear stress influences, through a series of cascade pathways, the cellular morphology, alignment, gene expression, and signalling pathways, all of which are essential for cell behaviour and function. For instance, with endothelial cells, shear stress triggers responses including modulation of cell proliferation, cell alignment in the direction of flow, expression of specific surface receptors involved in many physiological responses, and structural cytoskeleton remodelling.

Shear stress application is vital to enable *in vitro* model development of the structure and function of the vascular endothelium and other fluid-exposed tissues accurately. However, the intensity and duration of shear stress must be precisely controlled in MPS, as excessive shear can lead to cell damage, inflammation, or detachment from surfaces.

Advances in MPS design and microfluidic technology are enabling increasingly accurate control of shear stress, which is crucial for developing more predictive and physiologically relevant *in vitro* models for drug testing, disease modelling, and tissue engineering.

For this, MPS device development has been initially achieved using single glass then polymer devices, often made from Polydimethylsiloxane (commonly known as PDMS in English). In which, the polymer PDMS provides a gas permeable, transparent, and flexible device with microfluidic scale channels to provide laminar flow with shear stress application to the 3D cell culture models. While these MPS devices have meant greater control of the biophysical parameters for basic research, such devices are highly challenging to scale into high-throughput models that could be then integrated into existing industrial pipelines for drug development.

As a result, MPS device integration with established laboratory approaches, notably comprising a standardized multi-well plate comprising a standardized well, are becoming increasingly vital to enable use biological entities in pharmaceutical drug development assays. However, microfluidic channel integration within standardized multi-well plates remains highly challenging.

There is therefore a need for generating a sufficient homogenous laminar fluid flow within the well of a multi-well cell culture plate to mimic specific *in vivo* physiological features on the biological entity being cultured inside the well. In particular, this sufficient homogenous laminar fluid flow results in the application of a controlled shear stress at the level of the biological entity.

### Disclosure of the Invention

The invention fulfils this need by proposing a cell culture system comprises :
- a multi-well cell culture plate comprising at least one well having a predetermined longitudinal shape delimited by an inner peripheral edge and terminating in a bottom surface which is used for culture of a biological entity,
- a microfluidic device configured to be mounted on the multi-well cell culture plate to create at least one closed chamber in which a volume of liquid cell culture medium flows from a liquid cell culture medium inlet opening into the at least one closed chamber to a liquid cell culture medium outlet opening into the at least one closed chamber in order to culture the biological entity,
the microfluidic device comprises a manifold containing at least one nozzle having a predefined longitudinal shape extending along a longitudinal axis delimited by an outer peripheral edge and terminating in an insertion base substantially perpendicular to the longitudinal axis configured to be inserted into the at least one well to form the at least one closed chamber having a height defined between the insertion base of the at least one nozzle and the bottom surface of the at least one well, the at least one nozzle containing an inlet channel connected to the liquid cell culture medium inlet opening configured to inject a controlled volume of liquid cell culture into the at least one closed chamber, and an outlet channel connected to the liquid cell culture medium outlet opening configured to extract a part of the volume of liquid cell culture medium from the volume of liquid cell culture medium contained in the closed chamber.

Advantageously, the cell culture system allows to achieve a homogenous laminar flow, both speed and direction, of the volume of liquid cell culture medium flowing in the closed chamber. With this cell culture system, the shear stress applied to the biological entity can be controlled in function of the flow rate of the volume of liquid cell culture medium. In particular, this shear stress can be greater than 15 dyne/cm². This system also has the advantage of allowing any bubbles generated in the closed chamber to be evacuated, while preventing these bubbles from coming into contact with the biological entity so as to avoid potential deleterious effect of cells or tissues of the biological entity.

Moreover, the cell culture system can reduce the impact of gas exchange between the flowing volume of liquid cell culture medium inside the closed chamber and a surrounding atmospheric air. This is possible because this cell culture system minimizes the volume of atmospheric air that could potentially come into contact with the flowing volume of liquid cell culture medium and minimizes the air to liquid cell culture medium interface inside the at least one closed chamber so that to reduce the impact of the atmospheric air on liquid cell culture medium inside the closed chamber.

Furthermore, the cell culture system avoids any physical phenomenon that can compromise the optical imaging quality, i.e. the at least one nozzle doesn't cause shadow presence in imaging.

Advantageously, the invention includes the following features, alone or in combination:
- the at least one nozzle is in polymer, preferably a polymer chosen from polyethylene, a cyclic olefin copolymer, a polycarbonate, a polystyrene, or a mixture comprising at least one of these;
- the insertion base of the at least one nozzle and the bottom surface of the at least one well are configured to be substantially parallel when the microfluidic device is mounted on the multi-well, and the height of the at least one closed chamber defined between the insertion base of the at least one nozzle and the bottom surface of the at least one well is greater than 0,1 mm, preferably around 1 mm;
- the inlet channel and the outlet channel extend within the predefined longitudinal shape of the at least one nozzle, the inlet channel opening at the liquid cell culture medium inlet opening, and the outlet channel opening at the liquid cell culture medium outlet opening;
- the inlet channel and the outlet channel extend substantially parallel to the longitudinal axis of the predefined longitudinal shape of the at least one nozzle;
- the liquid cell culture medium inlet opening and the liquid cell culture medium outlet opening are each provided near the outer peripheral edge of the predefined longitudinal shape of the at least one nozzle and are opposed each other with respect to the longitudinal axis of the predefined longitudinal shape of the at least one nozzle;
- the liquid cell culture medium inlet opening opens out at least in part in a first straight section normal to the longitudinal axis along which the predefined longitudinal shape extends, a first truncated passage from the longitudinal edge of the predefined longitudinal shape is provided from the first straight section to the insertion base in order to allow the controlled volume of liquid cell culture to flow along a first part of the predefined longitudinal shape from the liquid cell culture medium inlet opening to the closed chamber; and
- the liquid cell culture medium outlet opening opens out at least in part in a second straight section normal to the longitudinal axis along which the predefined longitudinal shape extends, a second truncated passage from the longitudinal edge of the predefined longitudinal shape is provided from the second straight section to the insertion base in order to allow the part of the volume of liquid cell culture to flow along a second part of the predefined longitudinal shape from the closed chamber to the liquid cell culture medium outlet opening,
- the first truncated passage and/or the second truncated passage extend transversely to the longitudinal axis along which the predefined longitudinal shape extends from the outer peripheral edge over a distance of less than 3 mm,
- the distance separating the first straight section from the insertion base is greater than or equal to the distance separating the second straight section from the insertion base,
- the first straight section is at a distance between ¼ and ¾ of a predefined length of the predefined longitudinal shape from the insertion base and the second straight section is at a distance between 1/10 and ½ of the predefined length of the predefined longitudinal shape from the insertion base,
- the at least one nozzle is configured to be inserted into the at least one well in order to form a substantially constant air space less than 0,75 mm between the inner peripheral edge of the at least one well and the outer peripheral edge of the at least one nozzle,
- the manifold is connected to a connection orifice allowing the attachment of a fluid connector coming from a fluid flow controller,
- a sealing layer, in particular made in polymer, is disposed between the microfluidic device and the multi-well cell culture plate to hermetically seal said at least one closed chamber, the sealing layer having at least one orifice though which said at least one nozzle is configured to be inserted.
- wherein the microfluidic device further comprises a lid having at least one connection orifice to which at least one fluid connector of a fluid flow controller is configured to connect thereto, and a microfluidic panel defining at least one microfluidic channel, the microfluidic panel connecting the at least one well of the multi-well culture plate to the at least one nozzle of said manifold and to the corresponding at least one connection orifice of the lid, a microfluidic circuit being formed when the microfluidic device is combined with the multi-well cell culture plate.

Other purposes, characteristics and advantages of the invention will emerge more clearly upon reading the following description of a particular embodiment, provided as a simple non-restrictive example, in relation to the figures, among which:
[FIG. 1]: diagrammatically illustrates a view of the multi-well cell culture plate and the microfluidic device of a cell culture system according to an embodiment of the invention;
[FIG. 2]: diagrammatically illustrates a view of the multi-well cell culture plate and the microfluidic device of a cell culture system according to an embodiment of the invention;
[FIG. 3]: diagrammatically illustrates a view of a microfluidic device according to an embodiment of the invention, on a surface of which a sealing layer has been deposited;
[FIG. 4]: diagrammatically illustrates an exploded view of a microfluidic device according to an embodiment of the invention, on a surface of which a sealing layer has been deposited;
[FIG. 5]: diagrammatically illustrates a view of an assembled cell culture system according to an embodiment of the invention;
[FIG. 6]: diagrammatically illustrates a view of a nozzle of a cell culture system according to an embodiment of the invention;
[FIG. 7]: diagrammatically illustrates a cross-sectional view of a nozzle of a cell culture system according to an embodiment of the invention;
[FIG. 8]: diagrammatically illustrates a cross-sectional view of a nozzle of a cell culture system according to an embodiment of the invention in through which a volume of liquid cell culture flows;
[FIG. 9]: diagrammatically illustrates a cross-sectional view of a nozzle of a cell culture system according to an embodiment of the invention.

The general principle of the invention consists of confining and controlling the chemical, biochemical and physical properties in time and space, such that the shear stresses induced or the contact with the atmospheric air, of the biological environments in the devices such as for example microfluidic devices and in the cell culture plates containing several wells, also called multi-well culture plates, by means of a specially designed device. The invention is useful for performing controlled biological testing in the fields of discovery and pharmacological and biotechnological testing, controlled cell differentiation, therapeutic and cosmetic testing, research and testing of compounds and customised testing, as well as in all life sciences experiments requiring a precise control of the environmental conditions of the cells, tissues or organoids.

However, it should be noted that the invention, rather than being applied to a multi-well plate, can be used directly in a cell culture chamber having a circular geometry shape.

More specifically, the invention simultaneously proposes a cell culture system to achieve a homogenous laminar flow, both speed and direction, of a volume of liquid cell culture medium flowing in a closed chamber in which a biological entity is cultured. With this cell culture system, the shear stress applied to the biological entity can be controlled in function of the flow rate of the volume of liquid cell culture medium. In particular, this shear stress can be greater than 15 dyne/cm². This system also has the advantage of allowing any bubbles generated in the closed chamber to be evacuated, while preventing these bubbles from coming into contact with the biological entity so as to avoid potential deleterious effect of cells or tissues of the biological entity.

Moreover, the cell culture system can reduce the impact of gas exchange between the flowing volume of liquid cell culture medium inside the closed chamber and a surrounding atmospheric air. This is possible because this cell culture system minimizes the volume of atmospheric air that could potentially come into contact with the flowing volume of liquid cell culture medium and minimizes the air to liquid cell culture medium interface inside the at least one closed chamber so that to reduce the impact of the atmospheric air on liquid cell culture medium inside the closed chamber.

Subsequently, for the purposes of simplification, "cell culture" is understood to mean a non-organised or organised cell culture, namely a cell assembly such as for example tissues, spheroids or organoids. These cells can for example be from animals, such as humans, mice, or plants.

"Multi-well plate" is understood to mean standard plates comprising at least six wells in which a biological entity like specific nutrient culture medium, certain chemical or biochemical compounds, with the cell type studied is introduced, as well as cells to be cultured. For example, the multi-well plate can comprise 6, 12, 48 or 96 wells.

"Microfluidic" is understood to mean both microfluidic flow rate (between 1ul/min and 999ul/min) and milifluidic flow rate (between 1ml/min and 99ml/min). In particular, "microfluidic" is understood to mean the manipulation of reduced fluidic volumes (10 to 100's of µl) with channel diameters ranging from 5 to 500µm and "milifluidic" representing larger fluidic volumes (1ml and higher) with fluidic channel diameters ranging from above 500µm.

"Closed chamber" is understood to mean, for example, an isolated reservoir which could be connected to any device and more specifically to any microfluidic device for the purposes of the invention, and which can, if necessary, be associated with the fluid flow controller, or else the device according to the invention can be associated with a multi-well plate by means of an adaptive device designed for this purpose such as that described in this invention. The closed chamber is then formed in this case by the nozzle and the well of the multi-well cell culture plate when the microfluidic device is mounted on and seals the multi-well cell culture plate. For example, one of more isolated reservoir(s) forming a closed chamber can be mounted in series or in parallel with one or more devices according to the invention.

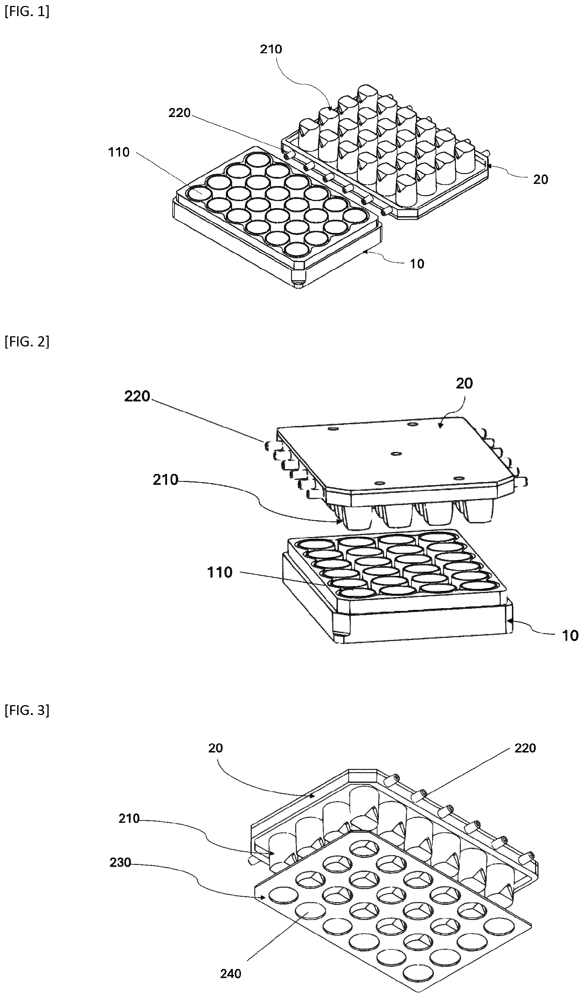

We now show, in relation to FIG. 1, FIG. 2, FIG. 3, FIG. 4 and FIG. 5, the structure of the cell culture system, according to an embodiment of the invention.

The cell culture system comprises a multi-well cell culture plate 10 and a microfluidic device 20.

The multi-well cell culture plate 10 with shape selected from among the cubic, rectangular shapes but preferably substantially parallel pipe-shaped, comprises a plurality of wells 110 having a predetermined longitudinal shape delimited by an inner peripheral edge and terminating in a bottom surface 111 which is used for culture of the biological entity.

It should be noted that, it is possible to have several wells 110 mounted in series and/or in parallel to each other. In other words, the multi-well cell culture plate 10 is configured to integrate one or more wells 110 or to adapt to one or more wells thus forming one or more closed chambers mounted in series and/or in parallel and in which it is possible to cultivate biological entities.

It should be noted that the biological entity cultured in a closed chamber can be identical or different from the one culture in another closed chamber.

The microfluidic device 20 with shape selected from among the cubic, rectangular shapes but preferably substantially parallel pipe-shaped corresponding to the shape of the multi-well cell culture plate 10, comprises a lid, and a part called a manifold, comprising a series of nozzles 210, also called orifices or ducts forming the base of this microfluidic device 20.

The lid has, on two opposite edges, one or more connection orifices 220, also called fluidic connection ducts (not shown), to which at least one fluid connector (not shown) of a fluid flow controller is connected thereto. This lid can be entirely opaque or, in a variant, allow the passage of light in order to obtain a real-time optical image of the biological entities.

In a variant, on the external top surface of the manifold, several zones (not shown) allow the passage of light and thus a visualisation by microscope or by other means necessary to obtain real-time images of the biological entities. The manifold preferably has a flat surface at least on its bottom surface 212. It can be manufactured with different materials including, but not limited to, polymers such as PMMA (poly(methyl methacrylate)), COP (cyclic olefin polymer), COC (Cyclic olefin copolymer) PS (polystyrene), PEEK (polyetheretherketone), or PC (polycarbonate).

The fluid flow controller is configured to inject, through the fluid inlet into the volume of liquid cell culture medium, a controlled volume of liquid into the closed chamber.

Moreover, the microfluidic device 20 further comprises a microfluidic panel 250 defining at least one microfluidic channel.

The microfluidic panel 250 connects each well 110 of the multi-well culture plate 10 to one of the plurality of nozzles 210 of the manifold. Furthermore, the microfluidic panel 250 connects the corresponding at least one connection orifice 220 of the lid to each nozzle 210. Also, a microfluidic circuit is formed when the microfluidic device 20 is combined with the multi-well cell culture plate 10.

In particular, the microfluidic panel 250 is then attached between flat surfaces of a bottom part of lid and a top part of a connecting plate 260 which guarantees the selective sealing of the microfluidic channels and structures. This microfluidic panel 250 is preferably made of double-sided adhesive tape, cut in order to define microfluidic channels connecting the different wells 110 of the multi-well culture plate 10 (not shown) to the nozzles 210 of the manifold and to the corresponding connection orifices 220 of the lid. Alternatively, the microfluidic panel 250 is constructed using a polymer bonding technique. This microfluidic panel 250 defines the fluidic connections of the microfluidic device 20.

In a variant, the microfluidic device 20 can be embodied in different ways, for example the microfluidic panel 250 is constructed in the lid, then bonded or connected to connecting plate 260 or to an attachment panel or directly to the manifold.

The connecting plate 260 has a substantially flat surface and facilitates the attachment of microfluidic panel 250 to the attachment panel and guarantees the selective sealing of the microfluidic channels. Hence, the lid can be attached by means of microfluidic panel 250, connecting plate 260 and attachment panel to the manifold, and consequently create the microfluidic device 20 to be subsequently mounted on the multi-well cell culture plate 10 in order to generate the cell culture system. This sandwich structure and its variants are embodied to guarantee the correct assembly of the different parts into a microfluidic structure allowing selective communication with the fluid flow controller, the movement of the fluids between the wells 110 of multi-well plate while avoiding undesirable contamination, and the external circulation of the liquids.

The surface of the connecting plate 260 is preferably transparent for an application where light must pass from the upper transparent zones of the lid to the bottom of the wells 110 of the multi-well plate for microscope imaging for example. The materials used to manufacture the connecting plate 260 are preferably chosen from among the COP or COC composites, glass or other transparent materials. The connecting plate 260 is traversed by a plurality of orifices enabling the microfluidic channels of microfluidic panel 250 to be connected to the corresponding channels of the manifold.

In a variant, the microfluidic device 20 can be embodied in different ways, for example a plate such as connecting plate 260 could be used between the lid and the microfluidic panel 250.

To attach the lid assembly, the microfluidic panel 250, the connecting plate 260 to the manifold, an attachment panel preferably made of double-sided adhesive tape is used. This attachment panel also allows good communication between the wells 110 of the multi-well culture plate 10 through a plurality of orifices connecting the microfluidic channels defined by microfluidic panel 250 to the nozzles 210 or to the orifices of the manifold.

Alternatively, this attachment panel can be eliminated and connecting plate 260 is then directly attached to the manifold. For example, the different elements are fixed together using a polymer bonding technique.

The manifold is a structure made of a flexible material, like for example an elastomer material such as polyurethane or silicone, defining microfluidic nozzles 210 reaching a defined depth in the multi-well culture plate 10 while sealing a fluidic circuit. This fluidic circuit is defined by the microfluidic channels of the microfluidic panel 250, orifices of connecting plate 260, orifices of attachment panel and the nozzles 210 of the manifold. The flexibility of the manifold facilitates the assembly of the device to the wells 110 of the multi-well culture plate 10 and guarantees the sealing of the resulting structure.

Alternatively, the manifold is a structure made of rigid material, like for example a polymer such as PMMA (poly(methyl methacrylate)), COP (cyclic olefin polymer), COC (Cyclic olefin copolymer), PS (polystyrene), PEEK (polyetheretherketone) or PC (polycarbonate), defining microfluidic nozzles 210 reaching a defined depth in the multi-well culture plate 10, while sealing a fluidic circuit. The device is assembled to the wells 110 of the multi-well culture plate 10 and guarantees the sealing of the resulting structure, for example by means of one or more O-ring seals or equivalent structure, or even by bonding.

The microfluidic panel 250 is then attached between the flat surfaces of the bottom part of the manifold and the top part of the connecting plate 260 which guarantees the selective sealing of the microfluidic channels and structures. This microfluidic panel 250 is preferably made of double-sided adhesive tape, cut in order to define microfluidic channels connecting the different wells 110 of the multi-well culture plate 10 (not shown) to the nozzles 210 of the manifold and to the corresponding connection orifices 220 the lid. Alternatively, the microfluidic panel 250 is constructed using a polymer bonding technique. This microfluidic panel 250 defines the fluidic connections of the microfluidic device 20.

Thus, the microfluidic device 20 is configured to be mounted on the multi-well cell culture plate 10 to create closed chambers in which a volume of liquid cell culture medium flows from a liquid cell culture medium inlet opening 215 into each of the closed chambers to a liquid cell culture medium outlet opening 216 into each of the closed chambers in order to culture the biological entity according to controlled conditions.

However, before this assembly, the biological entities are placed in the bottom surface 111 of the multi-well plate, along with the reagents, before closing it with the assembled cell culture system. The mediums, samples, cell cultures and reagents are placed in the wells 110 of the multi-well plate before the experiment (as is currently the case in pharmaceutical, biotechnological and biological laboratories) and remain isolated throughout its use after the assembly of the cell culture system according to the invention in order to form the closed chambers.

In a variant, one or more compound(s) are selectively perfused in independent individual wells 110.

When assembled, the microfluidic device 20 and the multi-well cell culture plate 10 are sealed and connected to the exterior only by the connecting orifices. The reagents and the biological entities are completely isolated from the exterior since the orifices are used to circulate the fluids, which are preferably prefiltered to prevent contamination.

The injecting fluid in the cell culture system is an injected controlled volume of liquid cell culture medium. The fluid contained in the closed chambers is a volume of liquid cell culture medium. The fluid extracted from the closed chambers is an extracted part of the volume of liquid cell culture medium.

According to the invention, the microfluidic device 20 comprises nozzles 210, if necessary with different heights, to control, in combination with the given fluid pressures and/or flows, which the volume of liquid cell culture medium that is perfused in the closed chambers which contain the biological entities. These nozzles 210 are designed to control, over time, the volume of liquid cell culture medium inside closed chambers for the biological entities. In particular, these nozzles 210 are configured to inject the controlled volume of liquid cell culture medium into the closed chambers, and to extract the extracted part of the volume of liquid cell culture medium from the closed chambers.

The nozzles 210 have a predefined longitudinal shape extending along a longitudinal axis delimited by an outer peripheral edge 211 and terminating in an insertion base 212 substantially perpendicular to the longitudinal axis. Each nozzle 210 is configured to be inserted into one well 110 to form the closed chamber having a height defined between the insertion base 212 of the nozzle 210 and the bottom surface 111 of the well 110. The nozzle 210 containing an inlet channel 213 connected to the liquid cell culture medium inlet opening 215 configured to inject a controlled volume of liquid cell culture into the closed chamber, and an outlet channel 214 connected to the liquid cell culture medium outlet opening 216 configured to extract a part of the volume of liquid cell culture medium from the volume of liquid cell culture medium contained in the closed chamber.

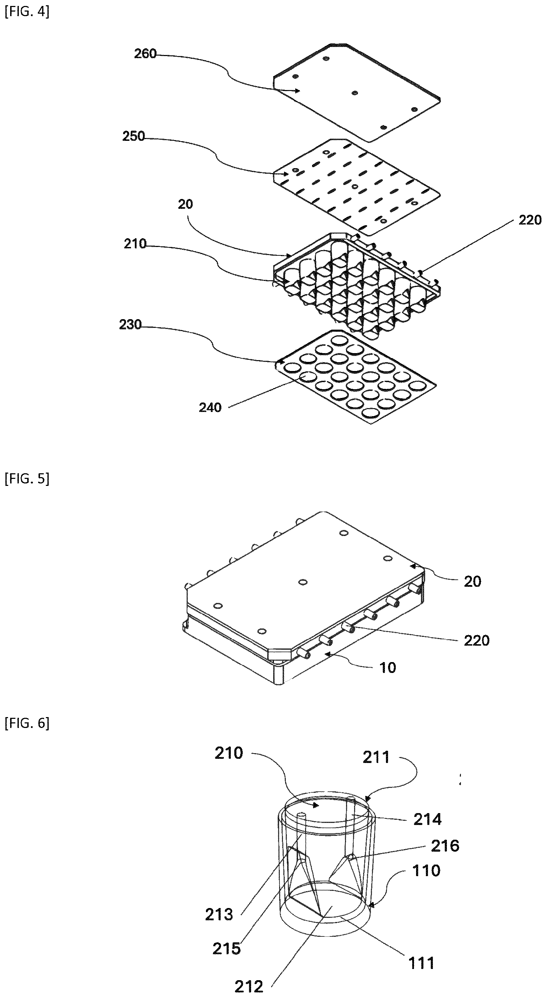

Advantageously, as represented in FIGS. 3 and 4, a sealing layer 230, in particular made in polymer, is disposed between the microfluidic device 20 and the multi-well cell culture plate 10 to hermetically seal the closed chamber. Moreover, the sealing layer 230 has at least one orifice 240 though which the nozzle 210 is configured to be inserted. Preferably, the thickness of the sealing layer 230 can be controlled in order to adjust the distance between the insertion base 212 of the nozzle 210 and the bottom surface 111 of the well 110 in which the nozzle 210 is placed.

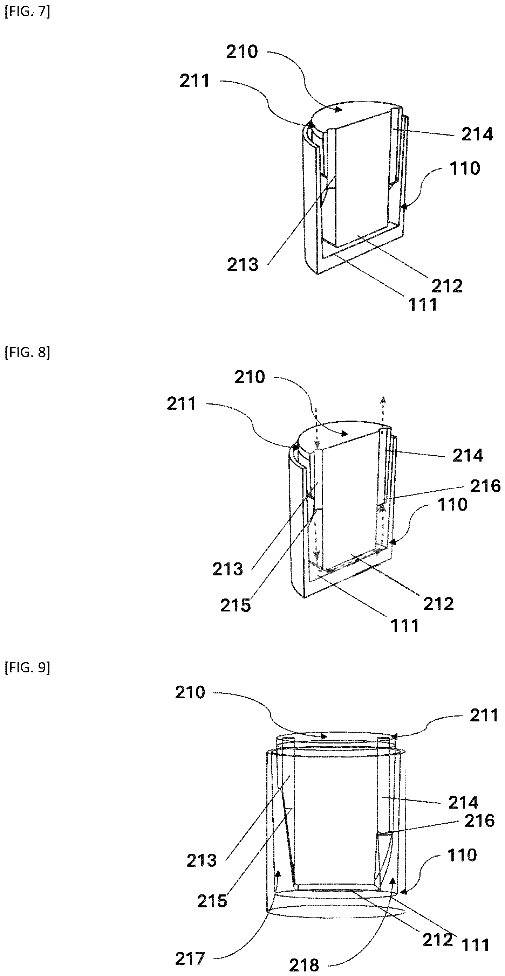

We now show, in relation to FIG. 6, FIG. 7, FIG. 8 and FIG. 9, a nozzle 210 contained by the manifold according to any embodiment in relation to FIGS. 1 to 5.

FIG. 6 shows a nozzle 210 having a predefined longitudinal shape extending along a longitudinal axis delimited by an outer peripheral edge 211 and terminating in the insertion base 212 substantially perpendicular to the longitudinal axis. This nozzle 210 is configured to be inserted into one well 110 of the multi-well cell culture plate 10 to form one closed chamber having a height defined between the insertion base 212 of the nozzle 210 and the bottom surface 111 of the well 110.

Preferably, the nozzle 210 is in polymer, preferably a polymer chosen from polyethylene, cyclic olefin copolymer (COC), polycarbonate (PC), polystyrene (PS), or a mixture comprising at least one of these. For example, the nozzle 210 can be made in a hydrophilic material.

Preferably, the insertion base 212 of the nozzle 210 and the bottom surface 111 of the well 110 are configured to be substantially parallel when the microfluidic device 20 is mounted on the multi-well. Furthermore, the height of the closed chamber defined between the insertion base 212 of the nozzle 210 and the bottom surface 111 of the well 110 is greater than 0,1 mm, preferably around 1 mm.

According to the invention, the nozzle 210 contains an inlet channel 213 connected to the liquid cell culture medium inlet opening 215 configured to inject a controlled volume of liquid cell culture into the closed chamber, and an outlet channel 214 connected to the liquid cell culture medium outlet opening 216 configured to extract a part of the volume of liquid cell culture medium from the volume of liquid cell culture medium contained in the closed chamber.

The cross-section of the conduits can take on any shape depending on the application required. For example, they can be disc-shaped, square, rectangular or any other shape. Preferably, as illustrated in figures, sections of these channels are disk-shaped.

Preferably, the inlet channel 213 and the outlet channel 214 extend within the predefined longitudinal shape of the nozzle 210. In particular, the inlet channel 213 opens at the liquid cell culture medium inlet opening 215, and the outlet channel 214 opens at the liquid cell culture medium outlet opening 216. This allows to provide the highest possible homogeneity of the speed of the injecting and extracting fluid speed and of the direction, notably when the injecting fluid comes into contact with biological entity.

Advantageously, the inlet channel 213 and the outlet channel 214 extend substantially parallel to the longitudinal axis of the predefined longitudinal shape of the at least one nozzle 210. This allows to further optimize the homogeneity of the speed and the direction of the injecting and extracting fluid.

Preferably, the liquid cell culture medium inlet opening 215 and the liquid cell culture medium outlet opening 216 are each provided near the outer peripheral edge 211 of the predefined longitudinal shape of the at least one nozzle 210 and are opposed to each other with respect to the longitudinal axis of the predefined longitudinal shape of the at least one nozzle 210. This allows to guarantee the largest possible surface area of the bottom surface 111 of the well 110 to be exposed to the perfused cell culture medium, without compromising the homogeneity of the injecting flow and/or the extracting flow. Moreover, this allows to maximise the closed chamber created between the insertion base 212 of the nozzle 210 and the biological entity, without compromising the homogeneity of the injecting flow and/or the extracting flow.

Advantageously, the liquid cell culture medium inlet opening 215 opens out at least in part in a first straight section normal to the longitudinal axis along which the predefined longitudinal shape extends. Moreover, a first truncated passage 217 from the longitudinal edge of the predefined longitudinal shape is provided from the first straight section to the insertion base 212. This allows the controlled volume of liquid cell culture to flow along a first part of the predefined longitudinal shape from the liquid cell culture medium inlet opening 215 to the closed chamber. This also ensures that the speed and flow of the injecting fluid directionality coming from the nozzle 210 into the closed chamber are uniform.

Preferably, the inlet channel 213 and outlet channel 214 of the nozzle 210 have a singular circular nozzle 210 form but can also be conical, slotted and angled or all predefined forms. The form is used to efficiently distribute the fluid from the inlet channel 213 of the nozzle 210 to the closed chamber and to the outlet channel 214.

Preferably, the first truncated passage 217 and/or the second truncated passage 218 extend transversely to the longitudinal axis along which the predefined longitudinal shape extends from the outer peripheral edge 211 over a distance of less than 3 mm in order to maximize the area of the closed chamber.

Preferably, the liquid cell culture medium outlet opening 216 opens out at least in part in a second straight section normal to the longitudinal axis along which the predefined longitudinal shape extends, a second truncated passage 218 from the longitudinal edge of the predefined longitudinal shape is provided from the second straight section to the insertion base 212 in order to allow the part of the volume of liquid cell culture to flow along a second part of the predefined longitudinal shape from the closed chamber to the liquid cell culture medium outlet opening 216.

Advantageously, the distance separating the first straight section from the insertion base 212 is greater than or equal to the distance separating the second straight section from the insertion base 212. This allows to have the highest area of continued and homogeneous injecting flow inside the closed chamber.

Preferably, the first straight section is at a distance between ¼ and ¾ of a predefined length of the predefined longitudinal shape from the insertion base 212 and the second straight section is at a distance between 1/10 and ½ of the predefined length of the predefined longitudinal shape from the insertion base 212. This makes it possible to homogenise the speed of the controlled volume of liquid cell culture injected into the closed chamber by the liquid cell culture medium inlet opening 215.

Advantageously, the at least one nozzle 210 is configured to be inserted into the at least one well 110 in order to form a substantially constant air space less than 0,75 mm between the inner peripheral edge of the at least one well 110 and the outer peripheral edge 211 of the at least one nozzle 210. This makes it possible to encourage the collection and the evacuation of air bubbles eventually contained in the closed chamber.

## Claims

1. A cell culture system comprises:
- a multi-well cell culture plate (10) comprising at least one well (110) having a predetermined longitudinal shape delimited by an inner peripheral edge and terminating in a bottom surface (111) which is used for culture of a biological entity,
- a microfluidic device (20) configured to be mounted on the multi-well cell culture plate (10) to create at least one closed chamber in which a volume of liquid cell culture medium flows from a liquid cell culture medium inlet opening (215) into the at least one closed chamber to a liquid cell culture medium outlet opening (216) into the at least one closed chamber in order to culture the biological entity,
the microfluidic device (20) comprises a manifold containing at least one nozzle (210) having a predefined longitudinal shape extending along a longitudinal axis delimited by an outer peripheral edge (211) and terminating in an insertion base (212) substantially perpendicular to the longitudinal axis configured to be inserted into the at least one well (110) to form the at least one closed chamber having a height defined between the insertion base (212) of the at least one nozzle (210) and the bottom surface (111) of the at least one well (110), the at least one nozzle (210) containing an inlet channel (213) connected to the liquid cell culture medium inlet opening (215) configured to inject a controlled volume of liquid cell culture into the at least one closed chamber, and an outlet channel (214) connected to the liquid cell culture medium outlet opening (216) configured to extract a part of the volume of liquid cell culture medium from the volume of liquid cell culture medium contained in the closed chamber.

2. The cell culture system according to claim 1, wherein the at least one nozzle (210) is in polymer, preferably a polymer chosen from polyethylene, a cyclic olefin copolymer, a polycarbonate, a polystyrene, or a mixture comprising at least one of these.

3. The cell culture system according to claims 1 to 2, wherein the insertion base (212) of the at least one nozzle (210) and the bottom surface (111) of the at least one well (110) are configured to be substantially parallel when the microfluidic device (20) is mounted on the multi-well, and the height of the at least one closed chamber defined between the insertion base (212) of the at least one nozzle (210) and the bottom surface (111) of the at least one well (110) is greater than 0,1 mm, preferably around 1 mm.

4. The cell culture system according to claims 1 to 3, wherein the inlet channel (213) and the outlet channel (214) extend within the predefined longitudinal shape of the at least one nozzle (210), the inlet channel (213) opening at the liquid cell culture medium inlet opening (215), and the outlet channel (214) opening at the liquid cell culture medium outlet opening (216).

5. The cell culture system according to claims 1 to 4, wherein the inlet channel (213) and the outlet channel (214) extend substantially parallel to the longitudinal axis of the predefined longitudinal shape of the at least one nozzle (210).

6. The cell culture system according to claims 1 to 5, wherein the liquid cell culture medium inlet opening (215) and the liquid cell culture medium outlet opening (216) are each provided near the outer peripheral edge (211) of the predefined longitudinal shape of the at least one nozzle (210) and are opposed each other with respect to the longitudinal axis of the predefined longitudinal shape of the at least one nozzle (210).

7. The cell culture system according to claim 6, wherein the liquid cell culture medium inlet opening (215) opens out at least in part in a first straight section normal to the longitudinal axis along which the predefined longitudinal shape extends, a first truncated passage (217) from the longitudinal edge of the predefined longitudinal shape is provided from the first straight section to the insertion base (212) in order to allow the controlled volume of liquid cell culture to flow along a first part of the predefined longitudinal shape from the liquid cell culture medium inlet opening (215) to the closed chamber.

8. The cell culture system according to claims 6 to 7, wherein the liquid cell culture medium outlet opening (216) opens out at least in part in a second straight section normal to the longitudinal axis along which the predefined longitudinal shape extends, a second truncated passage (218) from the longitudinal edge of the predefined longitudinal shape is provided from the second straight section to the insertion base (212) in order to allow the part of the volume of liquid cell culture to flow along a second part of the predefined longitudinal shape from the closed chamber to the liquid cell culture medium outlet opening (216).

9. The cell culture system according to claims 7 to 8, wherein the first truncated passage (217) and/or the second truncated passage (218) extend transversely to the longitudinal axis along which the predefined longitudinal shape extends from the outer peripheral edge (211) over a distance of less than 3 mm.

10. The cell culture system according to claims 6 to 9, wherein the distance separating the first straight section from the insertion base (212) is greater than or equal to the distance separating the second straight section from the insertion base (212).

11. The cell culture system according to claim 10, wherein the first straight section is at a distance between ¼ and ¾ of a predefined length of the predefined longitudinal shape from the insertion base (212) and the second straight section is at a distance between 1/10 and ½ of the predefined length of the predefined longitudinal shape from the insertion base (212).

12. The cell culture system according to claims 1 to 11, wherein the at least one nozzle (210) is configured to be inserted into the at least one well (110) in order to form a substantially constant air space less than 0,75 mm between the inner peripheral edge of the at least one well (110) and the outer peripheral edge (211) of the at least one nozzle (210).

13. The cell culture system according to claims 1 to 12, wherein the manifold is connected to a connection orifice (220) allowing the attachment of a fluid connector coming from a fluid flow controller.

14. The cell culture system according to claims 1 to 13, further comprising a sealing layer (230), in particular made in polymer, disposed between the microfluidic device (20) and the multi-well cell culture plate (10) to hermetically seal said at least one closed chamber, the sealing layer (230) having at least one orifice though which said at least one nozzle (210) is configured to be inserted.

15. The cell culture system according to claims 1 to 14, wherein the microfluidic device (20) further comprises a lid having at least one connection orifice (220) to which at least one fluid connector of a fluid flow controller is configured to connect thereto, and a microfluidic panel (250) defining at least one microfluidic channel,
the microfluidic panel (250) connecting the at least one well (110) of the multi-well culture plate (10) to the at least one nozzle (210) of said manifold and to the corresponding at least one connection orifice (220) of the lid, a microfluidic circuit being formed when the microfluidic device (20) is combined with the multi-well cell culture plate (10).
